Europäisches Patentamt

European Patent Office

Office européen des brevets

(11) Publication number: **0 372 088**
**A1**

# EUROPEAN PATENT APPLICATION
## published in accordance with Art. 158(3) EPC

(21) Application number: 89906445.5

(22) Date of filing: 02.06.89

(86) International application number:
PCT/JP89/00560

(87) International publication number:
WO 89/11882 (14.12.89 89/29)

(51) Int. Cl.5: **A61L 29/00, A61M 25/00**

(30) Priority: 06.06.88 JP 138756/88

(43) Date of publication of application:
13.06.90 Bulletin 90/24

(84) Designated Contracting States:
DE FR GB IT NL

(71) Applicant: SUMITOMO ELECTRIC INDUSTRIES, LTD
5-33, Kitahama 4-chome Chuo-ku
Osaka 541(JP)

(72) Inventor: KANAZAWA, Shinichi Osaka Works of Sumitomo Electr.
Ind., Ltd. 1-3, Shimaya 1-chome
Konohana-ku, Osaka-shi, Osaka 554(JP)
Inventor: YOTSUYA, Koro Osaka Works of Sumitomo Electr.
Ind., Ltd. 1-3, Shimaya 1-chome
Konohana-ku, Osaka-shi, Osaka 554(JP)
Inventor: SOGAWA, Ichiro Osaka Works of Sumitomo Electr.
Ind., Ltd. 1-3, Shimaya 1-chome
Konohana-ku, Osaka-shi, Osaka 554(JP)
Inventor: UEMIYA, Takafumi Osaka Works of Sumitomo Electr.
Ind., Ltd. 1-3, Shimaya 1-chome
Konohana-ku, Osaka-shi, Osaka 554(JP)
Inventor: NIWA, Shin-ichiro Osaka Works of Sumitomo Electr.
Ind., Ltd. 1-3, Shimaya 1-chome
Konohana-ku, Osaka-shi, Osaka 554(JP)

(74) Representative: Füchsle, Klaus, Dipl.-Ing.
Hoffmann . Eitle & Partner Patentanwälte
Arabellastrasse 4
D-8000 München 81(DE)

(54) BALLOON FOR CATHETER.

(57) Part or entire portion of a balloon membrane is made of a drug-supporting material capable of discharging gradually a drug, and/or a drug-containing solution is stored in the balloon and the balloon film

has pores for discharging the drug. This balloon for catheter is useful for prevention, diagnosis and remedy of various diseases including thrombosis.

Fig. 1

(a)

1

2

(b)

1

2

SPECIFICATION

CATHETER BALLOON

Technical field of the Invention

This invention relates to a catheter balloon which is used for medical treatments such as prevention, diagnosis, treatment of diseases, more particularly, to a catheter balloon which can provide a medicament to a living vessel wall.

Technical Background

In the treatment of cardiac thrombosis, blood vessel stenosis or obstruction, and the like, a balloon catheter having a balloon at the tip thereof has been used in order to expand the part of blood vessel stenosis or obstruction, or to destroy the lesion part by laser irradiation.

For instance, in order to detect or cure blood vessel stenosis or obstruction by the laser irradiation method, a balloon catheter is inserted to blood vessel and the balloon is inflated in the living vessel, where the blood vessel stenosis or obstruction may be detected, and the catheter is fixed within the living vessel inner wall, so that the inflated balloon is contacted to the inner wall of the living vessel, and consequently blood flow is stopped. Thereafter, an endoscope is inserted into the catheter to introduce the tip thereof to the detective position, and then, the absence or presence of blood vessel stenosis or obstruction is examined with sending a transparent flush liquid as a blood substitute into the detective position. When the blood vessel stenosis or obstruction is detected, the endoscopy is stopped, and then, the above-mentioned endoscope is replaced by a laser fiber to apply the laser to the part of the blood vessel stenosis or obstruction in order to destroy it optically or thermally.

However, in the above methods using a conventional balloon catheter, for example, when blood vessel is occluded by the balloon, vascular endothelial cells, which form the blood vessel inner wall, are easily destroyed or peeled off, because the balloon is directly contacted to the blood vessel inner wall. Especially, when the cells are peeled off, blood vessel stenosis or obstruction is easily reproduced at the peeling position, which rather causes appearance of a new lesion than curing.

Besides, the balloon rubs the blood vessel inner wall to stimulate it, hence, there is often occurred "Spasm" which is an intermittent convulsion of the blood vessel.

Under the circumstances, the present invention has been accomplished, and an object of the invention is to provide a catheter balloon which can safely be used for the medical treatment without occurrence of a new lesion after the medical treatment.

Disclosure of the Invention

In order to achieve the above object, the present inventors have intensively studied and have found to provide a medicament-supplying ability to the balloon per se.

An object of the invention is to provide an improved catheter balloon which can be used safely for the medical treatment of the living vessel without occurrence of any new lesion after the treatment. Another object of the invention is to provide a catheter balloon carrying a medicament in the balloon membrane or within the balloon and having an ability of sustained release of a medicament. A further object of the invention is to provide a catheter balloon having a plenty of pores on the balloon membrane so that the medicament contained within the balloon can be released gradually. These and other objects and advantages of the invention will be apparent to those skilled in the art from the following description.

The catheter balloon of this invention is characteristic in that a part or whole of the balloon membrane is composed of a medicament-carrying material which can release the carried medicament gradually, and other preferable embodiment of the catheter balloon of this invention is characterized by having a plenty of pores on the balloon membrane, from which the medicament contained within the balloon can be released out of the balloon.

According to the catheter balloon of this invention, when the balloon fixed to the catheter is inserted into the body cavity of the affected part and is inflated therein, the balloon is contacted directly to or approached nearby the inner wall of the body cavity and then the medicament carried by the balloon is gradually released to treat the affected part.

Besides, in the catheter balloon having a plenty of pores for releasing medicament, when the balloon fixed to the catheter is inserted into the body cavity of the affected part and then is inflated therein, the balloon is contacted directly to or approached nearby the inner wall of the body cavity and subsequently the medicament in the balloon is gradually released through the said pores to treat the affected part.

Brief Description of the Drawing

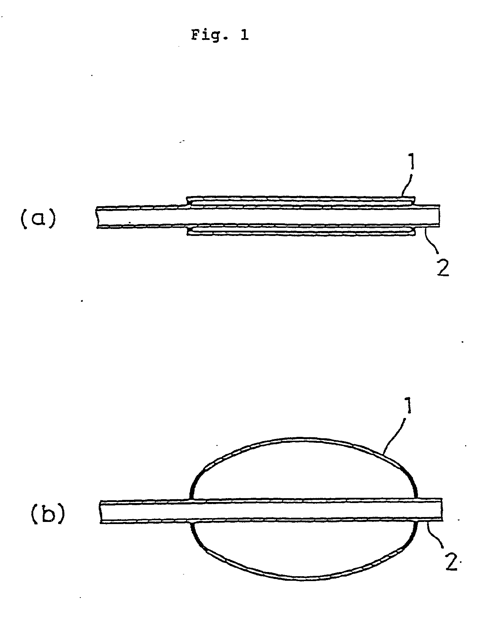

Fig. 1 (a) (b) are a schematic sectional view of a balloon catheter having the balloon of this invention.

Best Mode for Practicing the Invention

Referring to the attached figure, this invention will be illustrated in more detail hereinbelow.

Fig. 1 is an illustrative sectional view of the balloon catheter having the balloon of this invention, and said figure (a) shows the state of the catheter balloon before the balloon is inflated, and said fugure (b) shows the state of the catheter balloon after the balloon is inflated. In the figure, (2) is the catheter and around the tip of the catheter (2) there is the balloon (1) fixed.

During the inflation of the balloon (1), the part of the balloon membrane facing the cavity wall is composed of a material which can carry the medicament temporarily and release it gradually, such as a high molecular weight compound carrying the medicament by ion-bond or by hydrogen-bond, or porous silica mixed and kneaded with the medicament. And the fixed part or its neighborhood (in the figure, the parts marked in black) is composed of a material which is rich in elasticity in order to make it possible for the balloon (1) to expand, for example, an elastomer such as silicon rubber and the like.

The medicament to be carried includes heparin having an anti-thrombogenic activity and a vascular endothelial cells protecting activity; nitrate compounds such as nitroglycerin having a cardiac activity and a vasodilative activity (an activity of preventing vasoconstriction after treatment) and the like; β-blocker and calcium antagonists having a vasodilative activity; fibrinolytic agents having a thrombolytic activity, such as tissue plasminogen activator, urokinase, and the like.

The high molecular weight compound used for preparing the balloon membrane includes compounds having a low frictional characteristic in order not to injure the vascular endothelial cells, such as PTFE (polytetrafluoroethylene) or the elongated product thereof, polyvinyl chloride, polyethylene, the elongated product thereof and the like. Further, it may also include the above-mentioned elastic high molecular weight compounds being rich in elasticity such as silicon rubber, fluorinated elastomer (e.g., polyvinylidene fluoride, etc.), acrylelastomer, SBR, polychloroprene, polybutadiene, and the like.

The method of the medical treatment using the above-mentioned balloon catheter will be illustrated by referring to a case of the detection and treatment of blood vessel stenosis or obstruction.

First, the balloon catheter is inserted into the blood vessel and the balloon thereof is subjected to inflation in the living vessel where the blood vessel stenosis or obstruction may be detected, by which the balloon membrane is contacted to the blood vessel inner wall, and subsequently the catheter is fixed within the blood vessel inner wall and the blood flow is stopped. Further, an endoscope is inserted into the catheter to introduce the tip thereof into the detective position, and then, the absence or the presence of blood vessel stenosis or obstruction, and the exact position thereof are examined with sending a transparent flush liquid as a blood substitute into the detective position. When the blood vessel stenosis or obstruction is detected, the endoscopy is stopped, and the above-mentioned endoscope is replaced by a laser fiber, and the laser is applied to the part of the blood vessel stenosis or obstruction in order to destroy it optically or thermally.

The above-mentioned method for using the balloon catheter is not different from the conventional methods, but when the balloon catheter of this invention is used, even if the curing area by laser irradiation is injured by the contacting friction of the balloon, the re-production of lesion such as thrombus can effectively be inhibited by the medication with the balloon.

The balloon catheter illustrated in the above-mentioned examples consists of the elastic part made of a material having an elasticity and the part made of a material carrying and releasing the medicament, but the balloon of this invention should not be construed to be limited thereto, and it may include a balloon consisting of a material having at the same time the elasticity and the medicament-carrying and -releasing ability.

In the above-mentioned examples, the method of the balloon catheter in the treatment is illustrated by the case using laser irradiation, but the balloon catheter of this invention can also be used in other treatments, for example, the mechanical treatment, such as mechanical expansion of the vessel suffering from stenosis or obstruction, and the like. In this case, if there is an injury due to friction of inner cells during the procedure of stopping blood flow or expanding the part of blood vessel stenosis or obstruction, the medicament released from the balloon can heal and cure the injury immediately.

In other preferable embodiment of this invention, instead of carrying a medicament on the balloon membrane, or in addition thereto, the balloon catheter of this invention can contain a solution of the medicament as mentioned hereinbefore (e.g., physiological saline solution, etc.) in the balloon and can release the medicament gradually through a plenty of pores provided on the balloon membrane. The solution to be contained in the balloon may also be a physiological saline solution containing oxygen at a high rate, and in this case, the lack of oxygen due to the procedure of stopping blood flow can be prevented and also the blood vessel can be protected by releasing the medicament solution through the pores.

The pores are preferably provided in plenty on the balloon membrane, most preferably, uniformly whole of the balloon membrane. These pores can easily be obtained by using a commercially available porous material as a balloon material. The size of pores depends on the hydrophilicity or hydrophobicity of the balloon material, and it is usually preferred in the range of about 0.1 - 10 $\mu$m in hydrophilic material, and in the range of about 1 - 10 $\mu$m in hydrophobic material. In addition, when said medicament solution is contained in the balloon, the size of the pores may be provided so that the release of the medicament solution is effected by inflating the balloon inserted into the body cavity to expand pores thereof, or by controlling the inner pressure of the balloon inserted into the body cavity. Besides, the medicament solution may also be introduced into the balloon through the catheter after the catheter is inserted into the body cavity. In this case, there is an advantage that the medicament can be released continuously or intermittently over the required period of time.

Industrial Utilization and Effect of the Invention

The catheter balloon of this invention can be used in prevention, diagnosis and treatment of diseases such as various thrombosis, blood vessel stenosis or obstruction, and the like, and as is clear from the above-mentioned explanation, it is composed so as to be able to release the medicament by itself, hence, it shows various excellent effects, for example, it can effect the desired medical treatment safely, and also prevent occurrence of a new lesion after the treatment, and the like.

CLAIMS:

1. A catheter balloon which is characterized in that a part or whole of the balloon membrane is composed of a medicament-carrying material which can release the medicament gradually.

2. A catheter balloon of claim 1, wherein the balloon membrane is composed of a high molecular weight compound which carries the medicament.

3. A catheter balloon of claim 1, wherein the balloon membrane is composed of porous silica mixed and kneaded with the medicament.

4. A catheter balloon which is characterized in that the balloon membrane has a plenty of pores to release a medicament contained in the balloon gradually.

5. A catheter balloon of claim 4, wherein pores are uniformly provided whole of the balloon membrane.

EP 0 372 088 A1

Fig. 1

(a)

(b)

# INTERNATIONAL SEARCH REPORT

International Application No  PCT/JP89/00560

## I. CLASSIFICATION OF SUBJECT MATTER (if several classification symbols apply, indicate all) 6

According to International Patent Classification (IPC) or to both National Classification and IPC

Int. Cl⁴    A61L29/00, A61M25/00

## II. FIELDS SEARCHED

| Minimum Documentation Searched 7 | |
|---|---|
| Classification System | Classification Symbols |
| IPC | A61L29/00, A61M25/00 |

Documentation Searched other than Minimum Documentation
to the Extent that such Documents are included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | JP, A, 59-95058 (UNITIKA Ltd.) 31 May 1984 (31. 05. 84) (Family: none) | 1 - 3 |
| A | JP, A, 52-62996 (Akiyama Takashi) 24 May 1977 (24. 05. 77) (Family: none) | 1 - 3 |
| X | JP, A, 49-4390 (Salomon Hakim) 16 January 1974 (16. 01. 74) (Family: none) | 4 - 5 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 28, 1989 (28. 08. 89) | September 4, 1989 (04. 09. 89) |
| International Searching Authority | Signature of Authorized Officer |
| Japanese Patent Office | |

Form PCT/ISA/210 (second sheet) (January 1985)